# FASCICULE DE BREVET EUROPEEN

(11) **EP 0 386 216 B1**
(45) Date de publication et mention de la délivrance du brevet: **01.06.1994**
(21) Numéro de dépôt: 89910113.3
(22) Date de dépôt: 08.09.1989
(51) Int. Cl.: C08L 5/08, A61K 7/48

(54) **GEL AQUEUX A BASE D'ACIDE HYALURONIQUE ET D'ACIDE DESOXYRIBONUCLEIQUE UTILISABLE EN COSMETIQUE, ET PROCEDE DE PREPARATION**
WÄSSRIGES GEL VON HYALURONSÄURE UND DESOXYNIBONUKLEINSÄURE, VERWENDBAR FÜR KOSMETIK UND VERFAHREN ZU SEINER HERSTELLUNG
AQUEOUS GEL BASED ON HYALURONIC ACID AND DESOXYRIBONUCLEIC ACID USABLE IN COSMETICS, AND METHOD FOR ITS PREPARATION

(30) Priorité: 09.09.1988 FR 8811787
(43) Date de publication de la demande: 12.09.1990
(73) Titulaire: PIER AUGE, 46005 Châteauroux (FR)
(72) Inventeur: GARRIDO, Ricardo, F-36000 Châteauroux (FR)
(74) Mandataire: L'Helgoualch, Jean
(86) Numéro de dépôt international: FR8900452
(87) Numéro de publication internationale: WO9002774

(56) Documents cités:
- EP-A- 0 045 493
- CH-A- 652 600
- JP-A-59 134 706
- Chemical Abstracts, vol. 101, no. 22, 26 novembre 1984, (Columbus, Ohio, US), voir page 358, abrégé no. 197944u & JP, A, 59134706 (KOBAYASHI KOSE CO. LTD) 2 août 1984

## Description

L'invention concerne un nouveau gel aqueux à base d'acide hyaluronique et d'acide désoxyribonucléique hautement polymérisé, un procédé pour sa préparation, ainsi que son utilisation en cosmétique pour la préparation d'un masque de traitement de la peau.

L'acide hyaluronique est un polysaccharide à haut poids moléculaire qui constitue un composant important de la matrice extracellulaire des tissus conjonctifs, notamment dans la peau, les tendons, les cartilages et les muscles. L'acide hyaluronique peut être par exemple obtenu, sous forme de son sel de sodium, par extraction à partir de crêtes de coq, et l'on envisage sa production industrielle par fermentation bactérienne. Il présente une très grande aptitude à la rétention d'eau sous forme de solution ou de gel ayant des propriétés pseudo-plastiques et un écoulement visco-élastique.

L'acide hyaluronique et ses sels, en particulier son sel de sodium, sont utilisés dans des produits cosmétiques ayant un effet bénéfique sur la peau, notamment grace au fait que les solutions aqueuses d'acide hyaluronique ont un excellent effet hydratant.

D'autre part, on a proposé d'utiliser des gels réticulés à base d'acide hyaluronique pour retenir une substance ayant une activité biologique ou pharmacologique, et libérer progressivement cette substance, de manière contrôlée, au contact de la peau. De tels gels réticulés et leur utilisation sont, par exemple, décrits dans le brevet FR-A-2.574.414.

La demande de brevet JP-A-59.134706 décrit une composition pour l'hydratation de la peau contenant des acides nucléiques, un agent hydratant tel que l'acide hyaluronique, et divers principes actifs. Un gel destiné à la préparation d'un masque cosmétique, comprenant un alginate, un sel de métal bivalent, un phosphate, un agent tensio-actif anionique et une charge telle que SiO₂, ZnO, Al₂O₃, etc, est décrit dans la demande de brevet EP-A-0045493.

La présente invention a pour but de fournir un gel aqueux homogène comprenant d'une part de l'acide hyaluronique, et d'autre part une autre substance susceptible de présenter une activité bénéfique pour la peau, sous forme de gel aqueux, ce gel étant sous une forme appropriée à son application sur la peau, plus particulièrement en tant que masque de traitement cosmétique, et ayant la propriété de pouvoir être très rapidement durci, au moins en surface, de manière à pouvoir procurer un film cohérent par un processus simple et facile à régler, sans dégagement de chaleur ni libération ou mise en jeu de substance toxique.

A cet effet, le gel aqueux selon l'invention est caractérisé en ce qu'il contient un mélange homogène de:
a) 0,1 à 1% en poids du sel alcalin ou d'ammonium d'acide hyaluronique;
b) 1 à 3% en poids du sel minéral ou organique d'acide désoxyribonucléique à haut poids moléculaire;
c) 1 à 3% en poids d'au moins le polymère hydrophile ayant la propriété de former des gels aqueux filmogènes choisi parmi l'alginate de sodium et l'alginate d'ammonium.
d) une quantité suffisante d'eau pour compléter à 100%.

L'expression "ayant la propriété de former des gels aqueux filmogènes" se réfère à un polymère hydrophile capable de former un gel non réticulé ou une solution aqueuse ayant des propriétés pseudo-plastiques, ce gel ou cette solution pouvant être aisément et rapidement transformé en un film plastique cohérent par tout processus approprié, par exemple par insolubilisation ou réticulation.

Suivant une forme préférentielle de réalisation de l'invention, il est avantageux d'utiliser comme polymère hydrophile ayant la propriété de former des gels aqueux filmogènes, un alginate de sodium ou d'ammonium, et de préférence un alginate de sodium. L'alginate de sodium présente en outre l'avantage d'être hémostatique et de favoriser la cicatrisation des plaies ou coupures de la peau, ce qui améliore les qualités du gel de l'invention.

Il est également préférable, conformément à la présente invention, d'utiliser comme sel d'acide hyaluronique, un sel alcalin tel qu'un hyaluronate de sodium, et comme sel d'acide désoxyribonucléique, un sel de sodium, de calcium, de magnésium, de manganèse, de lysine ou d'arginine, et plus particulièrement un sel de sodium. Il est important, conformément à la présente invention, que l'acide désoxyribonucléique, sous forme de sel, soit hautement polymérisé.

L'acide désoxyribonucléique hautement polymérisé, ou acide désoxyribonucléique à haut poids moléculaire, est un produit disponible dans le commerce, obtenu à partir de cellules germinatives d'organismes eukaryotiques, provenant, par exemple, de laitance de poissons, par un procédé d'extraction adapté comprenant une opération de déprotéinisation de la matière de départ, en milieu de force ionique élevée, ainsi qu'une opération de floculation fibreuse en milieu alcoolique.

Le produit ainsi obtenu, constitué d'un sel minéral ou organique, de préférence un sel de sodium, d'acide désoxyribonucléique, se présente à l'état déshydraté sous forme de fibres de couleur blanc à blanc crème ayant une longueur de quelques centimètres.

Ce produit est soluble dans l'eau en formant des solutions aqueuses ou des gels qui se comportent comme un liquide non newtonien à écoulement pseudo-plastique. De telles solutions se distinguent radicalement des solutions aqueuses d'acide désoxyribonucléique ordinaire du commerce, de faible poids moléculaire, tel que par exemple l'acide désoxyribonucléique standard conforme à la pharmacopée française, dont l'écoulement est toujours de type newtonien.

Le sel de sodium d'acide désoxyribonucléique à haut poids moléculaire, ou hautement polymérisé, utilisable dans la présente invention est par exemple le sel commercialisé par la société Javenech (Fougères, France).

Suivant une forme particulièrement avantageuse de l'invention, le gel aqueux conforme à la présente invention est constitué d'un mélange homogène contenant:
a) 0,1 à 1% en poids d'un sel alcalin ou d'ammonium d'acide hyaluronique;
b) 1 à 3% en poids d'un sel minéral ou organique d'acide désoxyribonucléique à haut poids moléculaire;
c) 1 à 3% en poids d'au moins un polymère hydrophile ayant la propriété de former des gels aqueux filmogènes choisi parmi l'alginate de sodium et l'alginate d'aminonium,
d) une quantité suffisante d'eau pour compléter à 100%.

Il est préférable que le gel suivant la présente invention contienne en outre un conservateur qui peut être choisi parmi toutes les substances appropriées, acceptables en cosmétique, et par exemple l'acide déhydroacétique et ses sels, l'acide sorbique, un parabène, un conservateur connu sous l'une des dénominations commerciales Kathon CG, Sépicide HB, Germall II, Germaben II, Phénonip CLR, Nipastat, Bronopol, Méthoxyde sodé, Préserval, et Paridol. Ces conservateurs peuvent être utilisés isolément ou en combinaison de deux ou plusieurs, le cas échéant.

Suivant une forme de réalisation de la présente invention, le gel peut contenir un épaississant, qui permet alors de réduire la quantité de sel d'acide hyaluronique utilisé. Cet épaississant est utilisé de préférence en une quantité inférieure ou égale à 5% en poids par rapport au poids total du gel. Tout épaississant connu dans le commerce et acceptable en cosmétique peut être utilisé dans l'invention pourvu qu'il soit compatible avec le polymère hydrophile et notamment l'alginate de sodium, mais on utilise de préférence un dérivé d'acide polyacrylique, un dérivé cellulosique, des carraghénanes, une gomme de guar ou de caroube ou une gomme xanthane, et par exemple le produit dérivé d'acide polyacrylique commercialisé sous la marque Carbopol (Goodrich). Ces épaississants peuvent être utilisés isolément ou en combinaison, et par exemple on peut utiliser une combinaison carraghénane/caroube, xanthane/caroube, ou xanthane/guar.

Bien entendu, le gel aqueux conforme à la présente invention peut renfermer, outre les constituants principaux, les conservateurs et l'épaississant indiqués ci-dessus, tout ingrédient supplémentaire approprié, tel que par exemple une substance ayant une activité biologique ou pharmacologique, une substance ayant pour fonction d'optimaliser les propriétés physiques du gel, notamment ses caractéristiques rhéologiques, ou encore ses propriétés chimiques ou physico-chimiques, par exemple son pH, un parfum, un colorant, etc.

L'invention s'étend également à un procédé pour la préparation du gel aqueux décrit ci-dessus, qui se distingue en ce que l'on prépare un gel à propriétés pseudo-plastiques d'un sel alcalin ou d'un sel d'ammonium d'acide hyaluronique, on ajoute au moins un polymère hydrophile ayant la propriété de former des gels aqueux filmogènes, on prépare un gel d'un sel minéral ou organique d'acide désoxyribonucléique à haut poids moléculaire sous forme non-newtonienne, à propriétés pseudo-plastiques, que l'on ajoute audit gel de sel d'acide hyaluronique, et on mélange l'ensemble de manière à obtenir un gel homogène.

De préférence, le gel de sel d'acide hyaluronique est un gel préparé à partir d'une solution à 1% dans l'eau, et le gel d'acide désoxyribonucléique est un gel préparé à partir d'une solution à 5% dans l'eau, bien que ces valeurs puissent être modifiées en fonction des propriétés recherchées.

Le gel aqueux à base d'acide hyaluronique et d'acide désoxyribonucléique conforme à la présente invention peut être utilisé en cosmétique pour la réalisation d'un masque de traitement cosmétique en appliquant sur la peau à traiter au moins une couche de gel, en recouvrant ladite couche de gel par une gaze, puis par une deuxième couche de gel sur la gaze, en provoquant la transformation de la deuxième couche de gel en un film plastique, en enlevant ultérieurement le film plastique ainsi formé, puis la première couche de gel non transformée restant sur la peau.

Dans le cas ou le polymère hydrophile ayant la propriété de former des gels aqueux filmogènes est un alginate de métal alcalin ou d'ammonium, et en particulier un alginate de sodium, de préférence on provoque la transformation en film de la couche de gel contenant un alginate, par pulvérisation d'une solution aqueuse d'un sel alcalin ou alcalino-terreux acceptable en cosmétique sur toute la surface de la couche, et en laissant ensuite cette solution agir sur la couche de gel pendant un temps suffisant pour transformer intégralement ladite couche de gel en film plastique.

Le sel en solution aqueuse pulvérisé sur la surface du gel pour provoquer la formation du film, est de préférence le chlorure de calcium.

Les caractéristiques et avantages de la présente invention apparaitront plus en détail dans l'exemple suivant donné à titre non limitatif.

### EXEMPLE

On prépare un gel aqueux ayant la composition suivante à partir d'une solution aqueuse de hyaluronate de sodium à laquelle on ajoute un alginate de sodium, puis un gel aqueux à 5% en poids de sel de sodium d'acide désoxyribonucléique à haut poids moléculaire (ADN Intégral, Javenech), que l'on mélange (sauf indication contraire, les pourcentages et rapports sont indiqués en poids):

| | |
|---|---|
| Hyaluronate de sodium (gel aqueux à 1% en poids) | 77,8% |
| Gel d'acide désoxyribonucléique (ADN) à haut poids moléculaire (gel aqueux à 5% en poids de sel de sodium d'ADN) | 20,0% |
| Alginate de sodium (Protanal SF vendu par la société Protan A/S, Norvège) | 2,0% |
| Agent conservateur (acide sorbique) | 0,2% |

Ce gel peut être utilisé pour l'application d'un masque de traitement cosmétique sur le visage et le cou.

Avant l'application du masque sur le visage, on nettoie la peau au moyen d'une lotion ou d'une crème nettoyante comportant des microbilles.

On applique une première couche de gel sur le visage et le cou, et l'on effectue un massage favorisant un début de pénétration des principes actifs contenus dans le gel, dans les premières couches de l'épiderme. On applique ensuite une deuxième couche de gel puis on recouvre le visage et le cou d'une gaze qui doit rester collée au gel sur toute la surface à traiter. Finalement, on applique une mince deuxième couche de gel sur la gaze.

On vaporise ensuite soigneusement une solution aqueuse à 10% en poids de chlorure de calcium CaCl₂, finement pulvérisée, sur toute la surface du gel.

On laisse cette solution agir pendant environ 30 minutes, ce qui provoque la plastification pratiquement complète de la couche superficielle du masque, pour former un film plastique cohérent. Pendant ce temps, les principes actifs contenus dans la première couche de gel en contact avec la peau sont absorbés par les couches de l'épiderme.

Le film est ensuite enlevé, et le visage est nettoyé de manière habituelle.

Ce traitement permet de restaurer l'équilibre hydro-lipidique de la peau grace à l'effet d'hydratation très prononcé du gel.

On constate notamment les effets bénéfiques suivants sur la peau:
- puissant effet anti-rides;
- amélioration de la sécrétion sébacée et régulation du film hydro-lipidique superficiel;
- accroissement considérable de l'élasticité et de la fermeté des téguments;
- effet d'augmentation exceptionnel de l'éclat du teint;
- pouvoir de photo-protection très efficace;
- modifications structurelles favorables, décelables par examen approprié, des composants dermo-épidermiques.

Les essais effectués ont montré que le gel conforme à la présente invention décrit ci-dessus est d'une parfaite inocuité.

## Revendications

1. Gel aqueux utilisable en cosmétique pour la préparation d'un masque caractérisé en ce qu'il contient :
a) 0,1 à 1% en poids d'un sel alcalin ou un sel d'ammonium d'acide hyaluronique sous forme de gel aqueux;
b) 1 à 3% en poids d'un sel minéral ou organique d'acide désoxyribonucléique à haut poids moléculaire, dont les solutions aqueuses ou gels sont du type non newtonien à écoulement pseudo-plastique, sous forme de gel aqueux;
c) 1 à 3% en poids d'au moins un polymère hydrophile ayant la propriété de former des gels aqueux filmogènes choisi parmi l'alginate de sodium et l'alginate d'ammonium.
d) une quantité suffisante d'eau pour compléter à 100%.

2. Gel selon la revendication 1, caractérisé en ce que ledit polymère hydrophile ayant la propriété de former des gels aqueux filmogènes est un alginate de sodium.

3. Gel selon la revendication 1, caractérisé en ce que le sel minéral ou organique d'acide désoxyribonucléique est choisi parmi les sels de sodium, de calcium, de magnésium, de manganèse, de lysine et d'arginine.

4. Gel selon l'une quelconque des revendications précédentes, caractérisé en ce qu'il contient en outre un épaississant en une quantité inférieure ou égale à 5% en poids par rapport au poids total du gel.

5. Gel selon l'une quelconque des revendications précédentes, caractérisé en ce qu'il contient en outre un conservateur.

6. Gel selon la revendication 4, caractérisé en ce que l'épaississant est choisi parmi un dérivé d'acide polyacrylique, un dérivé cellulosique, des carraghénanes, une gomme de guar ou de caroube ou une gomme xanthane, isolément ou en combinaison.

7. Utilisation en cosmétique du gel selon l'une quelconque des revendications 1 à 6, caractérisée en ce que l'on réalise un masque de traitement cosmétique en appliquant sur la peau à traiter au moins une couche de gel, en recouvrant ladite couche de gel par une gaze, puis par une deuxième couche de gel sur la gaze, en provoquant la transformation de la deuxième couche de gel en un film plastique, en enlevant ultérieurement le film plastique ainsi formé, puis la première couche de gel non transformée restant sur la peau.

8. Utilisation en cosmétique selon la revendication 7, caractérisée en ce que l'on provoque la transformation en film de la couche de gel contenant un alginate, par pulvérisation d'une solution aqueuse d'un sel alcalin ou alcalino-terreux acceptable en cosmétique sur toute la surface de la couche, et en laissant ensuite cette solution agir sur la couche de gel pendant un temps suffisant pour transformer intégralement ladite couche de gel en film plastique.

9. Utilisation en cosmétique selon la revendication 8, caractérisée en ce que ledit sel acceptable en cosmétique est le chlorure de calcium.

10. Procédé de préparation d'un gel aqueux selon l'une quelconque des revendications 1 à 6, caractérisé en ce que l'on prépare un gel à propriétés pseudo-plastiques d'un sel alcalin ou d'un sel d'ammonium d'acide hyaluronique, on ajoute au moins un polymère hydrophile ayant la propriété de former des gels aqueux filmogènes, on prépare un gel d'un sel minéral ou organique d'acide désoxyribonucléique à haut poids moléculaire sous forme non-newtonienne, à propriétés pseudo-plastiques, que l'on ajoute audit gel de sel d'acide hyaluronique, et on mélange l'ensemble de manière à obtenir un gel homogène.

11. Procédé selon la revendication 10, caractérisé en ce que le gel de sel d'acide hyaluronique est un gel préparé à partir d'une solution à 1% dans l'eau, et le gel d'acide désoxyribonucléique est un gel préparé à partir d'une solution à 5% dans l'eau.

## Claims

1. Aqueous gel for use in cosmetics for the preparation of a mask, characterized in that it contains :
a) from 0.1 to 1% by weight of an akaline or ammonium salt of hyaluronic acid in the form of an aqueous gel;
b) from 1 to 3% by weight of a mineral or organic salt of deoxyribonucleic acid of high molecular weight, the aqueous solutions or gel thereof being of non newtonian type with pseudo-plastic flow properties, in the form of aqueous gel;
c) from 1 to 3% by weight of at least one hydrophilic polymer having the property of forming aqueous film-forming gels, selected from sodium alginate and ammonium alginate.
d) a sufficient quantity of water to complete to 100%.

2. A gel according to claim 1, characterized in that the said hydrophilic polymer having the property of forming aqueous film-forming gels is a sodium alginate.

3. A gel according to claim 1, characterized in that the mineral or organic salt of deoxyribonucleic acid is selected from the sodium, calcium, magnesium, manganese, lysine and arginine salts.

4. A gel according to any of the preceding claims, characterized in that it further contains a thickener in a quantity less than or equal to 5% by weight with respect to the total weight of the gel.

5. A gel according to any of the preceding claims, characterized in that it further contains a preservative.

6. A gel according to claim 4, characterized in that the thickener is selected from a polyacrylic acid derivative, a cellulosic derivative, carrageenans, a guar gum or a carob gum or an xanthan gum, alone or in combination.

7. Use in cosmetics of the gel according to any of claims 1 to 6, characterized in that a mask for cosmetic treatment is formed by applying, to the skin to be treated, at least one layer of gel, by covering the said layer of gel with a gauze, then with a second layer of gel on the gauze, by bringing about the transformation of the second layer of gel into a plastic film, by later removing the plastic film thus formed, then the non-transformed first layer of gel remaining on the skin.

8. Use in cosmetics according to claim 7, characterized in that the transformation into a film of the layer of gel containing an alignate is brought about by spraying an aqueous solution of an alkaline or alkaline-earth salt acceptable in cosmetics over the entire surface of the layer, and by then letting this solution act on the layer of gel for a time sufficient to entirely transform the said layer of gel into a plastic film.

9. Use in cosmetics according to claim 8, characterized in that the said salt acceptable in cosmetics is calcium chloride.

10. A process for the preparation of an aqueous gel according to any of claims 1 to 6, characterized in that a gel with pseudo-plastic properties is prepared from an alkaline salt or from an ammonium salt of hyaluronic acid, at last one hydrophilic polymer having the property of forming aqueous film-forming gels is added, a gel is prepared of a mineral or organic salt of high molecular weight deoxyribonucleic acid in a non-Newtonian form, having pseudo-plastic properties, which is added to the said salt of hyaluronic acid, and the whole is mixed in such a way as to obtain a homogeneous gel.

11. A process according to claim 10, characterized in that the gel of hyaluronic acid salt is a gel prepared from a 1% solution in water, and the gel of deoxyribonucleic acid is a gel prepared from a 5% solution in water.

## Patentansprüche

1. Wässeriges Gel, das in der Kosmetik zur Herstellung einer Maske einsetzbar ist, dadurch gekennzeichnet, daß es enthält:
a) 0,1 bis 1 Gew.-% eines Alkalisalzes oder eines Ammoniumsalzes von Hyaluronsäure in Form eines wässerigen Gels;
b) 1 bis 3 Gew.-% eines mineralischen oder organischen Salzes von Desoxyribonukleinsäure mit hohem Molekulargewicht, dessen wässerige Lösungen oder Gels vom nicht-Newton'schen Typ mit pseudoplastischem Fließverhalten sind, in Form eines wässerigen Gels;
c) 1 bis 3 Gew.-% zumindest eines hydrophilen Polymers, das die Eigenschaft besitzt, filmbildende wässerige Gels zu bilden und aus Natriumalginat und Ammoniumalginat ausgewählt ist;
d) eine Wassermenge, die ausreicht, um auf 100% aufzufüllen.

2. Gel nach Anspruch 1, dadurch gekennzeichnet, daß das genannte hydrophile Polymer die Eigenschaft hat, filmbildende wässerige Gels zu bilden und ein Natriumalginat ist.

3. Gel nach Anspruch 1, dadurch gekennzeichnet, daß das mineralische oder organische Salz von Desoxyribonukleinsäure aus den Natrium-, Kalzium-, Magnesium-, Mangan-, Lysin- und Argininsalzen ausgewählt ist.

4. Gel nach einem der vorhergehenden Ansprüche, dadurch gekennzeichnet, daß es außerdem ein Verdickungsmittel in einer Menge unter oder gleich 5 Gew.-%, bezogen auf das Gesamtgewicht des Gels, enthält.

5. Gel nach einem der vorhergehenden Ansprüche, dadurch gekennzeichnet, daß es außerdem ein Konservierungsmittel enthält.

6. Gel nach Anspruch 4, dadurch gekennzeichnet, daß das Verdickungsmittel aus einem Polyacrylsäurederivat, einem Zellulosederivat, Carrageenanen, einem Guar- oder Carubingummi oder einem Xanthangummi, allein oder als Kombination, ausgewählt ist.

7. Verwendung eines Gels nach einem der Ansprüche 1 bis 6 in der Kosmetik, dadurch gekennzeichnet, daß man daraus eine kosmetische Behandlungsmaske macht, indem man auf der zu behandelnden Haut zumindest eine Gelschicht aufträgt, die genannte Gelschicht mit einer Gaze bedeckt, gefolgt von einer zweiten Gelschicht auf der Gaze, die Umwandlung der zweiten Gelschicht in einen plastischen Film hervorruft, schließlich den so gebildeten plastischen Film abhebt, wobei dann die erste nicht umgewandelte Gelschicht auf der Haut verbleibt.

8. Verwendung in der Kosmetik nach Anspruch 7, dadurch gekennzeichnet, daß die Umwandlung der ein Alginat enthaltenden Gelschicht in einen Film durch Zerstäuben einer wässerigen Lösung eines in der Kosmetik einsetzbaren Alkali- oder Erdalkalisalzes auf der gesamten Oberfläche der Schicht hervorgerufen wird, und indem man diese Lösung dann für einen Zeitraum auf die Gelschicht einwirken läßt, die ausreicht, um die genannte Gelschicht vollständig in einen plastischen Film umzuwandeln.

9. Verwendung in der Kosmetik nach Anspruch 8, dadurch gekennzeichnet, daß das genannte in der Kosmetik einsetzbare Salz Kalziumchlorid ist.

10. Verfahren zur Herstellung eines wässerigen Gels nach einem der Ansprüche 1 bis 6, dadurch gekennzeichnet, daß ein Gel mit pseudoplastischen Eigenschaften aus einem Alkalisalz oder einem Ammoniumsalz von Hyaluronsäure hergestellt wird, zumindest ein hydrophiles Polymer zugegeben wird, das die Eigenschaft aufweist, filmbildende wässerige Gels zu bilden, ein Gel eines mineralischen oder organischen Salzes von Desoxyribonukleinsäure mit hohem Molekulargewicht in nicht-Newton'scher Form mit pseudoplastischen Eigenschaften hergestellt wird, es dem genannten Hyaluronsäuresalzgel zugegeben wird und alles so gemischt wird, daß man ein homogenes Gel erhält.

11. Verfahren nach Anspruch 10, dadurch gekennzeichnet, daß das Hyaluronsäuresalzgel ein Gel ist, das ausgehend von einer 1%igen Lösung in Wasser hergestellt wurde, und das Desoxyribonukleinsäuregel ein Gel ist, das ausgehend von einer 5%igen Lösung in Wasser hergestellt wurde.
